Europäisches Patentamt

**European Patent Office**

**Office européen des brevets**

(11) Publication number: **0 424 460 B1**

# EUROPEAN PATENT SPECIFICATION

(45) Date of publication of patent specification: **24.11.93**　(51) Int. Cl.⁵: **C07C 2/58**

(21) Application number: **89908651.6**

(22) Date of filing: **11.07.89**

(86) International application number:
**PCT/US89/03050**

(87) International publication number:
**WO 90/00534 (25.01.90 90/03)**

(54) **HETEROGENEOUS ISOPARAFFIN/OLEFIN ALKYLATION PROCESS.**

(30) Priority: **15.07.88 US 219129**
**15.07.88 US 219527**

(43) Date of publication of application:
**02.05.91 Bulletin 91/18**

(45) Publication of the grant of the patent:
**24.11.93 Bulletin 93/47**

(84) Designated Contracting States:
**BE DE FR GB IT NL**

(56) References cited:
**GB-A- 546 406        US-A- 2 450 764**
**US-A- 2 804 491      US-A- 2 939 890**
**US-A- 3 131 230      US-A- 3 800 003**
**US-A- 4 384 161**

(73) Proprietor: **MOBIL OIL CORPORATION**
**150 East 42nd Street**
**New York New York 10017(US)**

(72) Inventor: **CHOU, Tai-Sheng**
**207 Burd Street**
**Pennington, NJ 08534(US)**

Inventor: **HUSS, Albin, Jr.**
**51 Stirling Way**
**Chadds Ford, PA 19317(US)**
Inventor: **KENNEDY, Clinton, Robert**
**1116 St. Finnegan's**
**West Chester, PA 19382(US)**
Inventor: **KURTAS, Robert, Steve**
**3 Centauri Avenue**
**Sewell, NJ 08080(US)**
Inventor: **TABAK, Samuel, Allen**
**204 East Pine Street**
**Wenonah, NJ 08090(US)**

(74) Representative: **Colmer, Stephen Gary et al**
**Patent Department**
**c/o Mobil Services Company Limited**
**Mobil Court**
**3 Clements Inn**
**London WC2A 2EB (GB)**

**Description**

The present invention relates to a heterogeneous process for alkylating an isoparaffin with an olefin to provide an alkylate product useful as a high octane blending component in gasoline.

This invention results from a need to improve octane ratings for gasoline. Isoparaffin-olefin alkylation is a means to produce highly branched paraffins which can be blended into gasoline to effect the required octane improvement.

Alkylation is a reaction in which an alkyl group is added to an organic molecule. Thus, an isoparaffin can be reacted with an olefin to provide an isoparaffin of higher molecular weight. Industrially, the concept depends on the reaction of a $C_2$ to $C_5$ olefin with isobutane in the presence of an acidic catalyst producing a so-called alkylate. This is a very valuable blending component in the manufacture of gasolines because of its high octane rating.

Traditionally, the process in the industry includes the use of hydrofluoric acid or sulfuric acid as catalysts under controlled temperature conditions. Low temperatures are utilized in the sulfuric acid process to minimize the side reaction of olefin polymerization, and the acid strength is generally maintained at 88 to 94% by the continuous addition of fresh acid and the continuous withdrawal of spent acid. The hydrofluoric acid process is less temperature-sensitive and the acid is easily recovered and purified.

However, the hydrofluoric acid and sulfuric acid alkylation processes have inherent drawbacks including environmental concerns, acid consumption and sludge disposal. With increasing demands for octane and increasing environmental concerns, there is a need to develop an alkylation process based on a solid catalyst system.

U.S. Patent No. 2,804,491 relates to isoparaffin/olefin alkylation to make gasoline at temperatures between -20° and 150°F (-29 and 66°C) utilizing a two component catalyst comprising essentially excess $BF_3$ with a silica stabilized gel alumina.

U.S. Patent No. 4,384,161 describes a process of alkylating isoparaffins with olefins to provide alkylate using as a catalyst a large pore zeolite capable of absorbing 2,2,4-trimethylpentane and a Lewis acid such as boron trifluoride, antimony pentafluoride or aluminum trichloride. The use of a large pore zeolite in combination with the Lewis acid is reported to increase the activity and selectivity of the zeolite thereby effecting alkylation with high olefin space velocity and low isoparaffin/olefin ratio. According to the patent, problems arise in the use of solid catalysts in that they appear to age rapidly and cannot perform effectively at high olefin space velocity and the patent teaches the above solution to rectify the problem utilizing a zeolite type catalyst.

The present invention is based on the discovery that isoparaffin/olefin alkylation to produce high octane gasoline blending components can be effected with reduced catalyst aging problems compared to prior art processes by the use of a catalyst comprising a Lewis acid, such as $BF_3$, in combination with non-zeolite solid inorganic oxide, such as $SiO_2$ or $Al_2O_3$, and in the presence of a controlled amount of water.

Accordingly, the invention resides in a process for alkylating an isoparaffin containg up to 20 carbon atoms with an olefin containing from 2 to 12 carbon atoms comprising contacting the isoparaffin and olefin with a catalyst comprising a non-zeolitic solid inorganic oxide and a Lewis acid at a temperature of -40°C to 500°C and a pressure up to 35000 kPa (5000 psig), wherein the molar ratio of the isoparaffin to the olefin is from 1:1 to 50:1 and the contacting takes place in the presence of water.

The catalyst employed in the process of the invention comprises a Lewis acid and an inorganic oxide. A Lewis acid is generally considered to be a molecule which is capable of combining with another molecule or ion by forming a covalent chemical bond with two electrons from the second molecule or ion, that is to say, the Lewis acid is an electron acceptor. Examples of Lewis acids include boron trifluoride ($BF_3$), boron trichloride ($BCl_3$), antimony pentafluoride ($SbF_5$) and aluminum chloride ($AlCl_3$). The present invention contemplates the use of all Lewis acids, such as those set forth in "Friedel-Crafts and Related Reactions", Interscience Publishers, Chapter III and IV (1953), although boron trifluoride is preferred.

Useful inorganic oxides for the catalyst of the invention include alumina, silica, boria, oxides of phosphorus, titanium dioxide, zirconium dioxide, chromia, zinc oxide, magnesia, calcium oxide, silica-alumina, silica-magnesia, silica-alumina-magnesia, silica-alumina-zirconia, chromia-alumina, alumina-boria, silica-zirconia, and the various naturally occuring inorganic oxides of various states of purity such as bauxite, clay and diatomaeous earth. The preferred inorganic oxides are amorphous silicon dioxide and aluminum oxide.

The operating temperature of the alkylation process can extend over a fairly broad range, for example, from -40°C to 500°C but is preferably within the range of from about -40°C to 250°C and more preferably -40°C to 20°C. The practical upper operating temperature will often be dictated by the need to avoid an undue occurrence of undesirable side reactions.

The pressure used in the present process can also extend over a wide range, for example, from subatmospheric to 35000 kPa (5000 psig), preferably to 3600 kPa (500 psig).

Similarly the amount of catalyst used in the present process can be varied over relatively wide limits. In general, the amount of catalyst, as measured by the weight hourly space velocity of the olefin, can range from 0.01 to 100. It will be realized by those skilled in the art that the amount of catalyst selected for a particular reaction will be determined by several variables including the reactants involved as well as the nature of the catalyst and the operating conditions used.

The isoparaffin reactant used in the present alkylation process possesses up to 20 carbon atoms and preferably from 4 to 8 carbon atoms such as, for example, isobutane, 3-methylhexane, 2-methylbutane, 2,3-dimethylbutane and 2,4-dimethylhexane.

The olefin reactant used generally contains from 2 to 12 carbon atoms. Representative examples are ethylene, propylene, butene-1, butene-2, isobutylene, pentenes, hexenes, heptenes and octenes. Particularly preferred are $C_3$ and $C_4$ olefins and mixtures thereof. Conveniently the olefinic feed is first contacted with an isomerization catalyst to reduce its alpha-olefin content. Isomerization catalysts which can be used in the isomerization operation include catalysts which produce a shift of the double bond in 1-butene to a more central position in the hydrocarbon molecule to form 2-butene. Various conventional catalysts are suitable, including, for example, alumina, silica, zirconia, chromium oxide, boron oxide, thoria, magnesia, aluminum sulfate, and combinations thereof. Also suitable is a boron halide-modified metal oxide such as boron halide-modified substantially anhydrous or hydrous alumina. Thermal isomerization may be utilized, but suffers from the defect of producing excessive amount of side products. The isomerization step may also include a separation section to remove unconverted 1-butene, which can then be recycled to the isomerization reactor. Alkylation follows isomerization.

In general, the relative molar ratio between the isoparaffin reactant and the olefin alkylating agent is from 1:1 to 50:1, and preferably from 5:1 to 25:1.

A critical requirement of the present alkylation process is that water is introduced into the alkylation reactor, generally at a rate on average of 0.1 ppmw to 1 wt%, based upon total hydrocarbon feed rate, preferably at a rate of from 0.1 ppmw to 500 ppmw. The water can be supplied as such or as a feed material which provides water under the alkylation conditions selected. Suitable water-forming materials which can be introduced into the reactor without interfering with the desired alkylation include monohydric and dihydric alcohols which yield water upon undergoing dehydration. Of this group, particular preference is accorded the aliphatic alcohols, especially those containing 1 to 6 carbon atoms, for example, methanol, ethanol, isopropanol, t-butyl alcohol and isopentyl alcohol. The water and/or water-producing material can be added directly to the reactor, that is, as part of the feed and/or it can be incorporated in the catalyst either by direct contact or by exposing the catalyst to an atmosphere of water and/or water-forming material. The amount of water preintroduced into the catalyst ranges from 0.5 to 25 percent by weight of the catalyst, preferably from 1 to 10 percent.

The invention will now be particularly described with reference to the Examples and the accompanying drawings, in which:

Figure 1 shows the effect of $H_2O$ addition on catalyst aging as measured by $C_5$ + yield.

Figure 2 shows the effect of $H_2O$ addition on catalyst aging as measured by $C_9$ + yield.

EXAMPLE 1

This example illustrates the effect of $H_2O$ addition on the alkylate quality.

The feed was a 10:1 isobutane/olefin mixture in which the olefin component was a mixed $C_3 = /C_4 =$ fraction approximating that produced by an FCC unit and having the following distribution in weight%.

| Propylene | 42.5 |
|---|---|
| 1-butene | 13.7 |
| Cis + Trans-2-Butene | 28.2 |
| Isobutylene | 15.6 |

The non-zeolitic solid inorganic oxide used in this example was a commercially available amorphous $SiO_2$ (0.5 weight % $Al_2O_3$). The as-received material was calcined at 540°C (1000°F) and sized to 100/200 mesh before use in the alkylation reactor.

In a start-up procedure, 10 grams of catalyst was placed in the 300 ml autoclave reactor, and about 300 ml of isobutane was charged to fill the reactor. The resulting mixture was cooled to the desired temperature

3

with constant stirring at 1900 RPM and $BF_3$ gas was introduced into the reactor After $BF_3$ breakthrough was observed, the $BF_3$ flow rate was reduced to a level equivalent to 3 wt % of total hydrocarbon feed rate. At this point, the isobutane/olefin mixture was continuously fed into the reactor to initiate the catalytic alkylation. The operating conditions were 1135 kPa (150 psig), 20°C, 1900 RPM stirring rate, 1.2 WHSV based on olefin and 3.0 wt % $BF_3$ based on total hydrocarbon feed rate. The product was continuously withdrawn from the reactor, adjusted to atmospheric pressure via a back pressure regulator and then sent to a receiver which was kept at O°C. Periodically, the product was drained from the receiver and allowed to reach room temperature prior to anlaysis.

An on-line gas chromatograph coupled with an automatic sampling device was used to monitor the course of the alkylation reaction. All reported octane numbers were measured.

The resulting yield and octane data for the $BF_3/SiO_2$ catalyst system, summarized in Table 1, show a comparison between alkylation with and without added water. In case of water addition, water was added intermittently throughout the run at an average rate of about 100 ppmw based upon total hydrocarbon feed rate.

## TABLE 1

### THE EFFECT OF WATER ADDITION ON $BF_3$ PROMOTED ALKYLATION

| Catalyst System | $BF_3/SiO_2$ | $BF_3/SiO_2/H_2O$ |
|---|---|---|
| Yield, g $C_5+$/g Olefin Converted | 2.1 | 2.1 |
| **Yields in $C_5+$s, WT %** | | |
| $C_5$ | 3.4 | 2.7 |
| $C_6$ | 3.3 | 2.3 |
| $C_7$ | 29.1 | 27.1 |
| $C_8$ | 54.7 | 61.4 |
| $C_9+$ | 9.6 | 6.4 |
| RON+O | 91 | 91 |
| MON+O | 89 | 90 |

The results show that alkylation is essentially complete in both cases based upon the high $C_5 +$ yield per g of olefin converted. However, low level $H_2O$ addition substantially improved alkylate quality over $BF_3$/silica catalyst alone as seen by the increased research and motor octanes and reduced $C_9 +$ yield.

## EXAMPLE 2

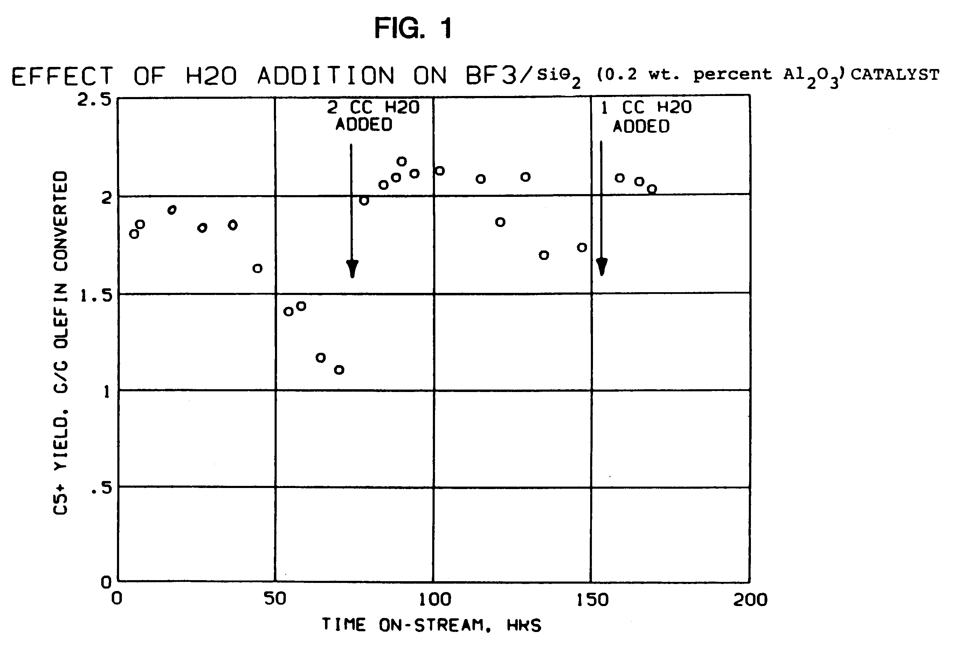

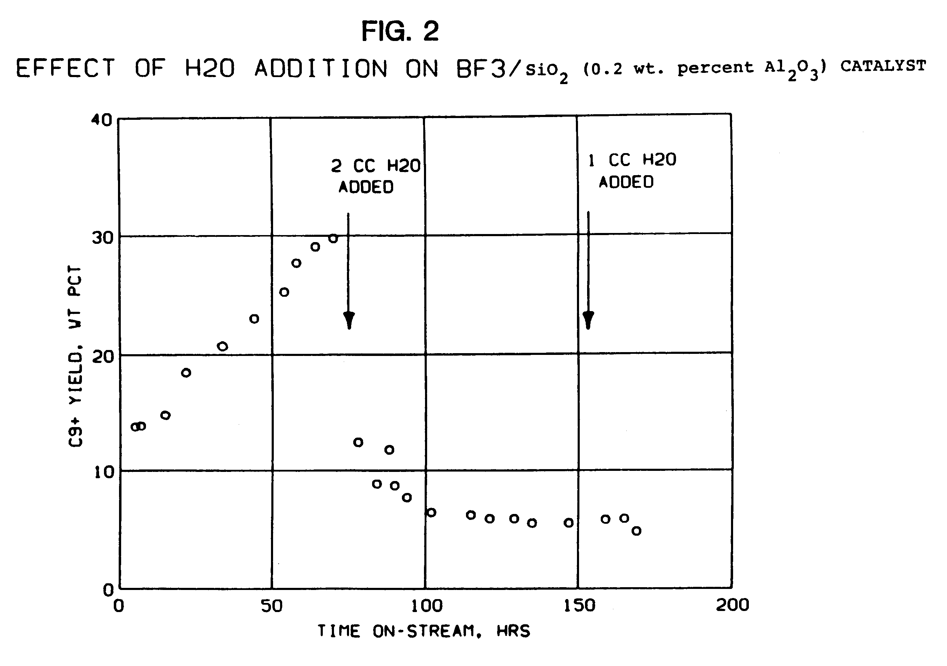

Example 1 was repeated but with the reaction temperature at O°C and the catalyst being $BF_3/SiO_2$ (0.2 wt% $Al_2O_3$) to show the effect of intermittent $H_2O$ addition on catalyst aging. The experiment was conducted without $H_2O$ addition until time on-stream was about 75 hours. At that point, 2cc $H_2O$ were added. Thereafter, 1 cc of $H_2O$ was added at about 155 hours. During the reaction the $C_5 +$ and $C_9 +$ yields were measured and the results are shown in Figure 1 and 2, respectively. As shown in Figure 1, the $C_5 +$ yield progressively degraded to about 1g $C_5 +$/g olefin converted until 2.0 cc of water was added at about 75 hours into the reaction. At this point, the $C_5 +$ yield increased dramatically to 2.1 g $C_5 +$/g olefin converted, indicating complete alkylation was restored. At about 155 hours into the reaction, an additional 1 cc of $H_2O$ was added to further extend the cycle length. Figure 2 shows that the addition of $H_2O$ inhibits $C_9 +$ formation, presumably by minimizing undesirable side reactions, e.g. polymerization. Thus, addition of $H_2O$ in an alkylation reaction using a $BF_3/SiO_2$ catalyst system significantly reduces catalyst aging.

## Claims

1. A process for alkylating an isoparaffin containing up to 20 carbon atoms with an olefin containing from 2 to 12 carbon atoms comprising contacting the isoparaffin and olefin with a catalyst comprising a non-zeolitic solid inorganic oxide and a Lewis acid at a temperature of -40 °C to 500 °C and a pressure up to 35000 kPa (5000 psig), wherein the molar ratio of the isoparaffin to the olefin is from 1:1 to 50:1 and the contacting takes place in the presence of water.

2.  The process of Claim 1, wherein the weight hourly space velocity of the olefin is from 0.01 to 100.

3.  The process of Claim 1, wherein the isoparaffin contains from 4 to 6 carbon atoms and the olefin contains from 2 to 6 carbon atoms.

4.  The process of Claim 1, wherein the isoparaffin is isobutane and the olefin is propylene and/or butenes.

5.  The process of Claim 1, wherein the Lewis acid is $BF_3$, $BCl_3$, $SbF_5$ and/or $AlCl_3$.

6.  The process of Claim 1, wherein the Lewis acid is $BF_3$.

7.  The process of Claim 1, wherein the inorganic oxide is $SiO_2$ or $Al_2O_3$.

8.  The process of Claim 1, wherein the catalyst is $BF_3/SiO_2$.

9.  The process of Claim 1, wherein the amount of water ranges from 0.1 ppmw to 1 weight percent based upon the total hydrocarbon feed rate.

10. The process of Claim 1, wherein reaction temperature is from -40 °C to 20 °C.

**Patentansprüche**

1.  Verfahren zur Alkylierung eines Isoparaffin, das bis zu 20 Kohlenstoffatome enthält, mit einem Olefin, das 2 bis 12 Kohlenstoffatome enthält, das den Kontakt des Isoparaffin und des Olefin mit einem Katalysator, der ein nichtzeolithisches festes anorganisches Oxid und eine Lewis-Säure umfaßt, bei einer Temperatur von -40 bis 500 °C und einem Druck bis zu 35000 kPa (5000 psig) umfaßt, wobei das Molverhältnis von Isoparaffin zu Olefin von 1:1 bis 50:1 beträgt und der Kontakt in Gegenwart von Wasser stattfindet.

2.  Verfahren nach Anspruch 1, worin die stündliche Gewichts-Raum-Geschwindigkeit des Olefin von 0,01 bis 100 beträgt.

3.  Verfahren nach Anspruch 1, worin das Isoparaffin von 4 bis 6 Kohlenstoffatome und das Olefin von 2 bis 6 Kohlenstoffatome enthält.

4.  Verfahren nach Anspruch 1, worin das Isoparaffin Isobutan und das Olefin Propylen und/oder Butene ist.

5.  Verfahren nach Anspruch 1, worin die Lewis-Säure $BF_3$, $BCl_3$, $SbF_5$ und/oder $AlCl_3$ ist.

6.  Verfahren nach Anspruch 1, worin die Lewis-Säure $BF_3$ ist.

7.  Verfahren nach Anspruch 1, worin das anorganische Oxid $SiO_2$ oder $Al_2O_3$ ist.

8.  Verfahren nach Anspruch 1, worin der Katalysator $BF_3/SiO_2$ ist.

9.  Verfahren nach Anspruch 1, worin die Wassermenge auf der Basis der gesamten Beschickungsmenge des Kohlenwasserstoffs im Bereich von 0,1 ppmw bis 1 Gew.-% liegt.

10. Verfahren nach Anspruch 1, worin die Reaktionstemperatur von -40 bis 20 °C beträgt.

**Revendications**

1. Procédé pour l'alkylation d'une isoparaffine contenant jusqu'à 20 atomes de carbone avec une oléfine contenant de 2 à 12 atomes de carbone comprenant la mise en contact d'une isoparaffine et d'une oléfine avec un catalyseur comprenant un oxyde inorganique solide non zéolitique et un acide de Lewis, à une température de -40 à 500°C et sous une pression allant jusqu'à 35000 kPa (5000 psig), dans lequel le rapport molaire isoparaffine/oléfine est compris entre 1/1 et 50/1 et la mise en contact a lieu en présence d'eau.

2. Procédé selon la revendication 1, dans lequel la vitesse spatiale horaire pondérale de l'oléfine est de 0,01 à 100.

3. Procédé selon la revendication 1, dans lequel l'isoparaffine contient de 4 à 6 atomes de carbone et l'oléfine contient de 2 à 6 atomes de carbone.

4. Procédé selon la revendication 1, dans lequel l'isoparaffine est l'isobutane et l'oléfine est le propylène et/ou les butènes.

5. Procédé selon la revendication 1, dans lequel l'acide de Lewis est $BF_3$, $BCl_3$, $SbF_5$ et/ou $AlCl_3$.

6. Procédé selon la revendication 1, dans lequel l'acide de Lewis est $BF_3$.

7. Procédé selon la revendication 1, dans lequel l'oxyde inorganique est $SiO_2$ ou $Al_2O_3$.

8. Procédé selon la revendication 1, dans lequel le catalyseur est $BF_3/SiO_2$.

9. Procédé selon la revendication 1, dans lequel la quantité d'eau est comprise entre 0,1 ppm et 1% en poids par rapport au débit d'alimentation total des hydrocarbures.

10. Procédé selon la revendication 1, dans lequel la température de réaction est comprise entre -40 et 20°C.

FIG. 1

EFFECT OF H2O ADDITION ON BF3/$SiO_2$ (0.2 wt. percent $Al_2O_3$) CATALYST

EP 0 424 460 B1

# FIG. 2

## EFFECT OF H2O ADDITION ON BF3/$SiO_2$ (0.2 wt. percent $Al_2O_3$) CATALYST

2 CC H2O ADDED

1 CC H2O ADDED

C9+ YIELD, WT PCT

TIME ON-STREAM, HRS